# EUROPEAN PATENT SPECIFICATION

(11) **EP 3 818 060 B1**
(45) Date of publication and mention of the grant of the patent: **22.02.2023**
(21) Application number: 19736624.8
(22) Date of filing: 27.06.2019
(51) Int. Cl.: C07D 471/04, A61K 31/437

(54) **NOVEL AMINO-IMIDAZOPYRIMIDINE DERIVATIVES AS JANUS KINASE INHIBITORS AND PHARMACEUTICAL USE THEREOF**
NEUARTIGE AMINO-IMIDAZOPYRIMIDIN-DERIVATE ALS JANUS-KINASE-INHIBITOREN UND PHARMAZEUTISCHE VERWENDUNG DAVON
NOUVEAUX DÉRIVÉS D'AMINO-IMIDAZOPYRIMIDINE UTILISÉS EN TANT QU'INHIBITEURS DE JANUS KINASE ET LEUR UTILISATION PHARMACEUTIQUE

(30) Priority: 06.07.2018 EP 18182186
(43) Date of publication of application: 12.05.2021
(73) Proprietor: LEO Pharma A/S, 2750 Ballerup (DK)
(72) Inventor: LARSEN, Jens C. Højland, 2750 Ballerup (DK); LARSEN, Mogens, 2750 Ballerup (DK); RITZEN, Andreas, 2750 Ballerup (DK)
(74) Representative: Zacco Denmark A/S
(86) International application number: PCT/EP2019/067127
(87) International publication number: WO 2020/007698

(56) References cited:
- EP-A1- 2 518 071
- WO-A1-2014/134750
- WO-A1-2018/130563

## Description

### FIELD OF THE INVENTION

This invention relates to compounds which are inhibitors of Janus kinases and derivatives thereof, to said compounds for use in therapy and to pharmaceutical compositions comprising said compounds.

### BACKGROUND OF THE INVENTION

This invention relates to novel compounds which are inhibitors of protein tyrosine kinases such as the Janus kinases (JAK1, JAK2, JAK3 and TYK2) and in particular Janus kinase 1 (JAK1).

Protein tyrosine kinases are a family of enzymes catalyzing the transfer of the terminal phosphate of adenosine triphosphate to tyrosine residues in protein substrates. Phosphorylation of tyrosine residues on protein substrates leads to transduction of intracellular signals which regulate a wide variety of processes such as cell growth differentiation and activation, metabolism, hematopoiesis, host defense and immunoregulation. As the elucidation of the molecular mechanisms in a number of inflammatory conditions and other disorders of the immune system (e.g. autoimmune diseases), highlighted the critical role of these intracellular signal pathways modulation of the activity of protein tyrosine kinases appears to be an attractive route to the management of inflammatory diseases. A large number of protein tyrosine kinases have been identified which may be receptor protein tyrosine kinases, e.g. the insulin receptor, or non-receptor protein tyrosine kinases.

The protein tyrosine kinases JAK1, JAK2, JAK3 and TYK2 selectively associate with the cytoplasmic domains of various cytokine receptor chains and have essential roles in cytokine-dependent regulation of tissue homeostasis, initiation of innate immunity, shaping adaptive immune responses and inflammatory processes. They are critical in signal transduction in response to their activation via tyrosine phosphorylation by stimulation of cytokine receptors. (1) Schindler C. et al. JAK-STAT signaling: from interferons to cytokines. J. Biol. Chem 2007; 282(28):20059; (2) O'Shea J.J. Targeting the Jak/STAT pathway for immunosuppression; Ann. Rheum. Dis. 2004; 63 Suppl 2:ii67; (3) Schindler C. Series introduction. JAK-STAT signaling in human disease; J. Clin. Invest. 2002; 109(9):1133); (4) O'Shea et. Al. Cell, Vol. 109, S121-S131, 2002; (5) Schwartz D.M. et al. Nat. Rev. Rheumatol., 2016; 12(1): 25-36; (6) O'Shea et al. New. Eng. J. Med. 2013; 368(2): 161-170.

While JAK1, JAK2 and TYK2 are ubiquitously expressed JAK3 is predominantly expressed in hematopoietic cells.

JAK1 plays a critical role in mediation of biological responses and JAK1 is widely expressed and associated with several major cytokine receptor families. It is involved in signaling by members of the IL-2 receptor γ subunit family (IL-2, IL-4, IL-7R, IL-9R, IL-15R and IL-21R), the IL-4 receptor family (IL-4R, IL-13R), the gp130 receptor family and class II cytokine receptors comprising of IL-10 receptor family and both type I and type II IFN receptor family.

JAK2 is implicated in signaling by several single chain receptors (including Epo-R, GHR, PRL-R), the IL-3 receptor family, the gp130 receptor family, the IL-12 receptor family (IL-12 and IL-23) and some Class II receptor cytokine family. Thus, JAK2 plays a critical role in transducing signals for Epo, IL-3, GM-CSF, IL-5 and IFNy. JAK2 knockout mice exhibit an embryonic lethal phenotype.

JAK3 is involved in signal transduction by receptors that employ the common gamma chain of the type I cytokine receptor family also known as IL-2 receptor family (e.g. IL-2, IL-4, IL-7, IL-9, IL-15 and IL-21). XSCID patient populations have been identified with reduced levels of JAK3 protein or with genetic defects to the common gamma chain, suggesting that immune suppression should result from blocking signaling through the JAK3 pathway. Animal studies have suggested that JAK3 not only plays a critical role in B and T lymphocyte maturation, but that JAK3 is constitutively required to maintain T cell function. Modulation of immune activity through this novel mechanism can prove useful in the treatment of T cell proliferative disorders such as immune system diseases, in particular autoimmune diseases.

TYK2 is implicated in type I interferons, IL-6, IL-10, IL-12 and IL-23 signaling. A human patient with a TYK2 deficiency has been described and this patient had a primary immunodeficiency disorder characterized as a hyper-IgE-like syndrome with many opportunistic infections by virus, bacteria and fungi. Because IL-23 has been found to play an important role in many chronic inflammatory conditions, a TYK2 inhibitor could conceivably be very effective in treating diseased influenced by IL-23.

Anemia and neutropenia may be related to inhibition of EPO and GM-CSF respectively, since the biological effect by these two cytokines apparently depends exclusively on JAK2 activation. Similarly, IL-12 and IL-23 are involved in engaging innate and adaptive immune defense to viruses, bacteria, and fungi. Because these cytokines bind to receptors that recruit JAK2 and TYK2 in their signaling cascade it is conceivable that a selective JAK1 inhibitor will not affect their biological activity and thus have a safer profile compared to compounds which inhibit JAK1, JAK2, JAK3 and TYK2.

Activation of JAK lead to the activation of STAT molecules and thus to the elicitation of JAK/STAT signaling pathway, which is highly regulated by phosphorylation events. Activation of STAT molecules is considered a valid pharmaco-dynamic marker for JAK activity and the activity of specific JAK molecules can be assessed by the level of preferential recruited active STAT molecule.

In particular, the receptor of IL-4 expressed by immune cells is constituted by two different chains, the ligand high affinity and signal transducer IL-4Ra and commonγ chain, activating JAK1 and JAK3 respectively upon ligand binding, which leads to the recruitment and activation of STAT6. Similarly, the IL-6 receptor is a heterodimer receptor formed by the IL-6 high affinity receptor chain (IL-6Ra) and the signal transducer glycoprotein 130 (gp130) chain to which JAK1 preferentially associates. The gp130 chain activates JAK1 and STAT3 signaling pathway upon ligand binding. Therefore, to investigate the activity of JAK1, the level of active STAT6 or STAT3 can be assessed in immune cells after stimulation with either IL-4 or IL-6, respectively. Furthermore, the receptor for erythropoietin (EPOR) is a homodimer receptor constituted by two identical receptor chains. Therefore, the EPOR chain is both high affinity ligand binding and signal transducer chain and activates only the associated JAK2 molecule upon ligand binding, leading to the recruitment and activation of STATS. Receptor for GM-CSF is a heterodimer receptor constituted by the GM-CSF high affinity receptor chain (GM-CSFRo) and the signal transducer chain (GM-CSFRβ), to which JAK2 specifically associates. Upon ligand binding, association of α and β receptor chains results in the activation of JAK2 and STAT5 signaling pathway. Therefore, to investigate the activity of JAK2, the level of active STAT5 can be assessed in immune cells after stimulation with either GM-CSF or erythropoietin (EPO).

Inhibitors of the Janus kinases are expected to show utility in the treatment of inflammatory and non-infectious autoimmune diseases wherein these kinases are involved. Recently the pan-JAK inhibitors Tofacitinib and Ruxolitinib have been launched for the treatment of rheumatoid arthritis and myelofibrosis, respectively.

Hence, JAK inhibitors may furthermore be useful in the treatment of diseases related to activity of Janus kinases, including, for example skin diseases like psoriasis, atopic dermatitis, scleroderma, rosacea, skin cancers, dermatitia, dermatitis herpetiformis, dermatomyositis, vitiligo, alopecia areata, contact dermatitis, eczema, xerosis, ichthyosis, urticaria and chronic idiophatic pruritus; respiratory diseases like asthma, chronic obstructive pulmonary disease, pulmonary fibrosis, cystic fibrosis, rhinitis, bronchiolitis, byssinosis, pneumoconiosis, bronchiectasis, hypersensitivity pneumonitis, lung cancers, mesothelioma and sarcoidosis; gastrointestinal diseases like inflammatory bowel disease, ulcerative colitis, Crohn's disease, retroperitoneal fibrosis, celiac disease and cancers; eye diseases like myasthenia gravis, Sjögren's syndrome, conjunctivitis, scleritis, uveitis, dry eye syndrome, keratitis, iritis; systemic indications like lupus, multiple sclerosis, rheumatoid arthritis, type I diabetes and complications from diabetes, cancers, ankylosing spondylitis and psoriatic arthritis; as well as other autoimmune diseases and indications where immunosuppression would be desirable for example in organ transplantation.

WO2013007768 discloses Tricyclic Heterocyclic Compounds, Compositions and Methods of use thereof as JAK Inhibitors.

WO2013007765 discloses Fused Tricyclic Compounds for use as Inhibitors of Janus Kinases

WO2011086053 discloses Tricyclic Heterocyclic Compounds, Compositions and Methods of use thereof.

EP2518071 discloses imidazo-pyridines as PI3 kinase inhibitors. There remains a need for new compounds which effectively and selectively inhibit specific JAK enzymes, in particular inhibitors which selectively inhibit JAK1 vs. JAK2 to reduce adverse effects without affecting the overall anti-inflammatory efficacy.

### SUMMARY OF THE INVENTION

Compounds of the present invention exhibit inhibitory activity on the Janus kinases; and in particular compounds of the invention exhibit inhibitory activity on JAK1. Thus compounds of the present invention show JAK kinase inhibitory selectivity; particularly the compounds show inhibitory selectivity of JAK1 vs. JAK2. It follows that compounds of the present invention may also show inhibitory selectivity of STAT6 or STAT3 vs. STAT5.

Accordingly, the present invention relates to a compound according to formula (I) 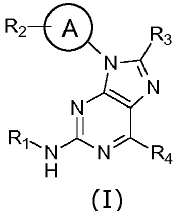 wherein
A represents C₆-cycloalkyl;
R₁ represents C₁-alkyl, wherein said C₁-alkyl is optionally substituted with one or more substituents selected from the group consisting of deuterium;
R₂ represents C₁-alkyl, wherein said C₁-alkyl is substituted with a substituent selected from R₅, and wherein said C₁-alkyl is optionally substituted with one or more deuterium;
R₃ represents C₂-alkyl, wherein said C₂-alkyl is substituted with a substituent selected from R₆ and wherein said C₂-alkyl is optionally substituted with one or more deuterium;
R₄ represents hydrogen or deuterium;
R₅ represents cyano;
R₆ represents hydroxyl;
or pharmaceutically acceptable salts, hydrates or solvates thereof.

In another aspect, the invention relates to a pharmaceutical composition comprising a compound of general formula (I) as defined herein together with a pharmaceutically acceptable vehicle or excipient or pharmaceutically acceptable carrier(s), optionally together with one or more other therapeutically active compound(s).

In yet another aspect, the invention relates to a compound according to general formula (I) as defined herein for use as a medicament.

In yet another aspect, the invention relates to a compound according to general formula (I) as defined herein for use in the prophylaxis and/or treatment of diseases of the immune system such as autoimmune diseases, or of diseases related to deregulation of the immune system.

### DETAILED DESCRIPTION OF THE INVENTION

### Definitions

The term "(Cₐ-C_{b})alkyl" is intended to indicate a radical obtained when one hydrogen atom is removed from a branched or linear hydrocarbon. Said alkyl comprises 1-6 preferably 1-4, such as 1-3, such as 2-3 or such as 1-2 carbon atoms. The term includes the subclasses normal alkyl (n-alkyl), secondary and tertiary alkyl, such as methyl, ethyl, n-propyl, isopropyl, n-butyl, isobutyl, sec.-butyl, tert.-butyl, n-pentyl, isopentyl, neopentyl, n-hexyl and isohexyl. The number of carbon atoms in "alkyl" is indicated by the prefix "(Cₐ-C_{b})", wherein a is the minimum number and b is the maximum number of carbons in the hydrocarbon radical. Thus, for example (C₁-C₄)alkyl is intended to indicate an alkyl radical comprising from 1 to 4 carbon atoms. C₁-alkyl is intended to indicate an alkyl radical comprising 1 carbon atom, such as methyl. C₂-alkyl is intended to indicate an alkyl radical comprising 2 carbon atoms, such as ethyl.

The term "cyano" is intended to indicate a -CN group attached to the parent molecular moiety through the carbon atom.

The term "(Cₐ-C_{b})cycloalkyl" is intended to indicate a saturated cycloalkane hydrocarbon radical, including polycyclic radicals such as bicyclic or tricyclic radicals, comprising 3-8 carbon atoms, such as 5-8 carbon atoms, such as 5-7 carbon atoms, such as 5-6 carbon atoms, such as 3-6 carbon atoms, such as 3-5 carbon atoms or such as 3-4 carbon atoms, such as 6 carbon atoms, e.g. cyclopropyl, cyclobutyl, cyclopentyl, cyclohexyl, or cycloheptyl, adamantly and cubanyl. The number of carbon atoms in "cycloalkyl" is indicated by the prefix "(Cₐ-C_{b})", wherein a is the minimum number and b is the maximum number of carbons in the hydrocarbon radical. Thus, for example (C₅-C₈)cycloalkyl is intended to indicate a cycloalkyl radical comprising from 5 to 8 carbon atoms.

The term "halogen" is intended to indicate a substituent from the 7^{th} main group of the periodic table, such as fluoro, chloro, bromo and iodo.

The term "hydrocarbon radical" is intended to indicate a radical containing only hydrogen and carbon atoms, it may contain one or more double and/or triple carbon-carbon bonds, and it may comprise cyclic moieties in combination with branched or linear moieties. Said hydrocarbon comprises 1-10 carbon atoms, and preferably comprises 1-4, e.g. 1-3, e.g. 1-2 carbon atoms. The term includes alkyl, cycloalkyl and aryl, as indicated herein.

The number of carbon atoms in a hydrocarbon radical (e.g. alkyl and cycloalkyl) is indicated by the prefix "(Cₐ-C_{b})", wherein a is the minimum number and b is the maximum number of carbons in the hydrocarbon radical. Thus, for example (C₁-C₄)alkyl is intended to indicate an alkyl radical comprising from 1 to 4 carbon atoms, and (C₅-C₈)cycloalkyl is intended to indicate a cycloalkyl radical comprising from 5 to 8 carbon ring atoms.

The terms "hydroxy" or "hydroxyl" are intended to indicate an -OH group.

The term "hydroxy(Cₐ-C_{b})alkyl" is intended to indicate an (Cₐ-C_{b})alkyl group as defined above substituted with one or more hydroxy, e.g. hydroxymethyl, hydroxyethyl, hydroxypropyl.

When two or more of the above defined terms are used in combination, such as arylalkyl, cycloalkylalkyl and the like, it is to be understood that the first mentioned radical is a substituent on the latter mentioned radical, where the point of attachment to the parent molecular moiety is on the latter radical.

The group C(O) is intended to represent a carbonyl group (C=O)

If substituents are described as being independently selected from a group, each substituent is selected independent of the other. Each substituent may therefore be identical or different from the other substituent(s).

The term "optionally substituted" means "unsubstituted or substituted", and therefore the general formulas described herein encompasses compounds containing the specified optional substituent(s) as well as compounds that do not contain the optional substituent(s).

The term "pharmaceutically acceptable salt" is intended to indicate salts prepared by reacting a compound of formula (I), which comprise a basic moiety, with a suitable inorganic or organic acid, such as hydrochloric, hydrobromic, hydroiodic, sulfuric, nitric, phosphoric, formic, acetic, 2,2-dichloroaetic, adipic, ascorbic, L-aspartic, L-glutamic, galactaric, lactic, maleic, L-malic, phthalic, citric, propionic, benzoic, glutaric, gluconic, D-glucuronic, methanesulfonic, salicylic, succinic, malonic, tartaric, benzenesulfonic, ethane-1,2-disulfonic, 2-hydroxy ethanesulfonic acid, toluenesulfonic, sulfamic or fumaric acid. Further examples of pharmaceutical acceptable salts are listed in Berge, S.M.; J. Pharm. Sci.; (1977), 66(1), 1-19, which is incorporated herein by reference.

The term "solvate" is intended to indicate a species formed by interaction between a compound, e.g. a compound of formula (I), and a solvent, e.g. alcohol, glycerol or water, wherein said species are in a crystalline form or in an amorphous form. When water is the solvent, said species is referred to as a hydrate.

The term "treatment" as used herein means the management and care of a patient for the purpose of combating a disease, disorder or condition. The term is intended to include the delaying of the progression of the disease, disorder or condition, the amelioration, alleviation or relief of symptoms and complications, and/or the cure or elimination of the disease, disorder or condition. The term includes prevention of the condition, wherein prevention is to be understood as the management and care of a patient for the purpose of combating the disease, condition or disorder and includes the administration of the active compounds to prevent the onset of the symptoms or complications. Nonetheless, prophylactic (preventive) and therapeutic (curative) treatments are two separate aspects.

Unless otherwise indicated, all exact values provided herein are representative of corresponding approximate values, e.g. exact exemplary values provided with respect to a particular measurement can be considered to also provide a corresponding approximate measurement, modified by "about" where appropriate.

All references, including publications, patent applications and patents, cited herein are hereby incorporated by reference in their entirety and to the same extent as if each reference were individually and specifically indicated to be incorporated by reference, regardless of any separately provided incorporation of particular documents made elsewhere herein.

### Embodiments of the invention

An embodiment of the invention provides a compound according to formula (I) above wherein 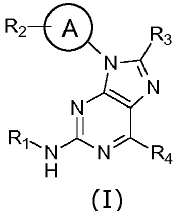
A represents C₆-cycloalkyl;
R₁ represents C₁-alkyl, wherein said C₁-alkyl is optionally substituted with one or more substituents selected from the group consisting of deuterium;
R₂ represents C₁-alkyl, wherein said C₁-alkyl is substituted with a substituent selected from R₅;
R₃ represents C₂-alkyl, wherein said C₂-alkyl is substituted with a substituent selected from R₆;
R₄ represents hydrogen;
R₅ represents cyano;
R₆ represents hydroxyl;
or pharmaceutically acceptable salts, hydrates or solvates thereof.

An embodiment of the invention provides a compound according to formula (I) above of formula (Ia) below

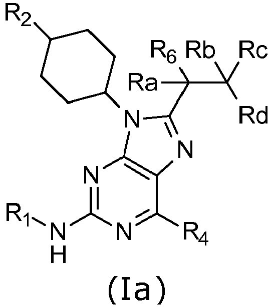

Wherein
R₁ represents C₁-alkyl, wherein said C₁-alkyl is optionally substituted with one or more substituents selected from the group consisting of deuterium;
R₂ represents C₁-alkyl, wherein said C₁-alkyl is substituted with a substituent selected from R₅ and wherein said C₁-alkyl is optionally substituted with one or more deuterium;
R₄ represents hydrogen or deuterium;
R₅ represents cyano;
R₆ represents hydroxyl;
Rₐ, R_{b}, R_{c} and R_{d} each independently are selected from hydrogen or deuterium;
or pharmaceutically acceptable salts, hydrates or solvates thereof.

In an embodiment the invention provides a compound of general formula (I); wherein formula (I) is general formula (Ib)

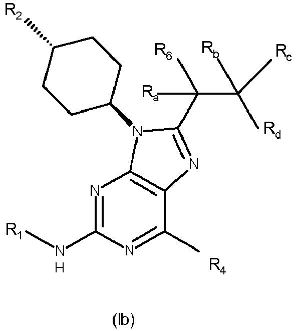

Wherein R₁, R₂, R₄ and R₆ are as defined above and wherein Ra, Rb, Rc and Rd each independently are selected from hydrogen and deuterium; or pharmaceutically acceptable salts, hydrates or solvates thereof.

In an embodiment the invention provides a compound of general formula (I); wherein formula (I) is general formula (Ic)

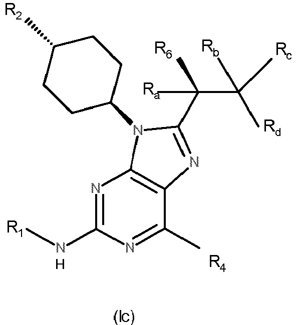

Wherein R₁, R₂, R₄ and R₆ are as defined above and wherein Ra, Rb, Rc and Rd each independently are selected from hydrogen and deuterium;or pharmaceutically acceptable salts, hydrates or solvates thereof.

In an embodiment the invention provides a compound of general formula (I); wherein formula (I) is general formula (Id)

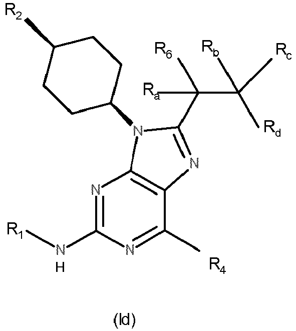

Wherein R₁, R₂, R₄ and R₆ are as defined above and wherein Ra, Rb, Rc and Rd each independently are selected from hydrogen and deuterium; or pharmaceutically acceptable salts, hydrates or solvates thereof.

An embodiment of the invention provides a compound of formula (I), said compound being selected from
2-[*trans*-4-[8-[(1*R*)-1-Hydroxyethyl]-2-(methylamino)purin-9-yl]cyclohexyl]acetonitrile,
2-[*trans*-4-[8-(1-Hydroxyethyl)-2-(methylamino)purin-9-yl]cyclohexyl]acetonitrile
or pharmaceutically acceptable salts, hydrates or solvates thereof.

An embodiment of the invention provides a compound of formula (I), said compound being selected from
2-[*trans*-4-[8-[(1*R*)-1-Hydroxyethyl]-2-(methylamino)purin-9-yl]cyclohexyl]acetonitrile,
2-[*trans*-4-[8-(1-Hydroxyethyl)-2-(methylamino)purin-9-yl]cyclohexyl]acetonitrile,
2-[*trans*-4-[8-[(1*R*)-1-Hydroxyethyl]-2-(trideuteriomethylamino)purin-9-yl]cyclohexyl]acetonitrile
or pharmaceutically acceptable salts, hydrates or solvates thereof.

An embodiment of the invention provides a compound of formula (I), said compound being selected from
2-[*trans*-4-[8-[(1*R*)-1-Hydroxyethyl]-2-(methylamino)purin-9-yl]cyclohexyl]acetonitrile,
or pharmaceutically acceptable salts, hydrates or solvates thereof.

Any combination of two or more embodiments described herein is considered within the scope of the present invention.

The compounds of formula (I) or pharmaceutically acceptable salts, hydrates or solvates thereof may be obtained in crystalline form either directly by concentration from an organic solvent or by crystallisation or recrystallisation from an organic solvent or mixture of said solvent and a cosolvent that may be organic or inorganic, such as water. The crystals may be isolated in essentially solvent-free form or as a solvate, such as a hydrate. The invention covers all crystalline forms, such as polymorphs and pseudopolymorphs, and also mixtures thereof.

Compounds of formula (I) may or may not comprise asymmetrically substituted (chiral) carbon atoms which give rise to the existence of isomeric forms, e.g. enantiomers and possibly diastereomers. The present invention relates to all such isomers, either in optically pure form or as mixtures thereof (e.g. racemic mixtures or partially purified optical mixtures). Pure stereoisomeric forms of the compounds and the intermediates of this invention may be obtained by the application of procedures known in the art. The various isomeric forms may be separated by physical separation methods such as selective crystallization and chromatographic techniques, e.g. high pressure liquid chromatography using chiral stationary phases. Enantiomers may be separated from each other by selective crystallization of their diastereomeric salts which may be formed with optically active amines, such as I-ephedrine, or with optically active acids. Optically purified compounds may subsequently be liberated from said purified diastereomeric salts. Enantiomers may also be resolved by the formation of diastereomeric derivatives. Alternatively, enantiomers may be separated by chromatographic techniques using chiral stationary phases. Pure stereoisomeric forms may also be derived from the corresponding pure stereoisomeric forms of the appropriate starting materials. If a specific stereoisomer is desired, said compound may be synthesized by stereoselective or stereospecific methods of preparation, and/or by employing chiral pure starting materials. Furthermore, when a double bond or a fully or partially saturated ring system is present in the molecule geometric isomers may be formed. It is intended that any geometric isomer, as separated, pure or partially purified geometric isomers or mixtures thereof are included within the scope of the invention.

In the compounds of general formula (I), the atoms may exhibit their natural isotopic abundances, or one or more of the atoms may be artificially enriched in a particular isotope having the same atomic number, but an atomic mass or mass number different from the atomic mass or mass number found in nature. The present invention is meant to include all suitable isotopic variations of the compounds of general formula (I). For example, different isotopic forms of hydrogen include ¹H, ²H and ³H and different isotopic forms of carbon include ¹²C, ¹³C and ¹⁴C, different isotopic forms of nitrogen include ¹⁴N, and ¹⁵N. Enriching for deuterium (²H) may for example increase in-vivo half-life or reduce dosage regiments, or may provide a compound useful as a standard for characterization of biological samples. Isotopically enriched compounds within general formula (I) can be prepared by conventional techniques well known to a person skilled in the art or by processes analogous to those described in the general procedures and examples herein using appropriate isotopically enriched reagents and/or intermediates.

In one or more embodiments of the present invention, the compounds of formula I as defined above are useful in therapy and in particular useful for treatment of for example skin diseases like proliferative and inflammatory skin disorders, psoriasis, atopic dermatitis, scleroderma, rosacea, skin cancers, dermatis, dermatitis herpetiformis, dermatomyositis, vitiligo, alopecia areata, contact dermatitis, eczema, xerosis, ichthyosis, urticaria and chronic idiophatic pruritus; respiratory diseases like asthma, chronic obstructive pulmonary disease, pulmonary fibrosis, cystic fibrosis, rhinitis, bronchiolitis, byssinosis, pneumoconiosis, bronchiectasis, hypersensitivity pneumonitis, lung cancers, mesothelioma and sarcoidosis; gastrointestinal diseases like inflammatory bowel disease, ulcerative colitis, Crohn's disease, retroperitoneal fibrosis, celiac disease and cancers; eye diseases like myasthenia gravis, Sjögren's syndrome, conjunctivitis, scleritis, uveitis, dry eye syndrome, keratitis, iritis; systemic indications like lupus, multiple sclerosis, rheumatoid arthritis, type I diabetes and complications from diabetes, cancers, ankylosing spondylitis and psoriatic arthritis; as well as other autoimmune diseases and indications where immunosuppression would be desirable for example in organ transplantation.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

In an embodiment the invention provides compounds of formula I as defined above for use in the prophylaxis and/or treatment of psoriasis or atopic dermatitis.

In an embodiment the invention provides a method of preventing, treating or ameliorating diseases of the immune system, such as autoimmune diseases, the method comprising administering to a person suffering from at least one of said diseases an effective amount of one or more compounds according to general formula I above optionally together with a pharmaceutically acceptable carrier or one or more excipients, optionally in combination with other therapeutically active compounds.

In an embodiment the invention provides a method of preventing, treating or ameliorating psoriasis or atopic dermatitis the method comprising administering to a person suffering from at least one of said diseases an effective amount of one or more compounds according to general formula I above optionally together with a pharmaceutically acceptable carrier or one or more excipients, optionally in combination with other therapeutically active compounds.

In an embodiment the invention provides a compound according to formula I for use in the manufacture of a medicament for the prophylaxis and/or treatment of diseases of the immune system, such as autoimmune disease, such as psoriasis or atopic dermatitis. In one or more embodiments of the present invention, the compounds of formula I as defined above are useful as an anti-inflammatory agent capable of modulating the activity of a protein tyrosine kinase of the JAK family of protein tyrosine kinases, such as JAK1, JAK2, JAK3 or TYK2 protein tyrosine kinases.

In one or more embodiment the invention provides a compound according to general formula (I) for use in the treatment of a disease, which disease is responsive to the inhibition JAK1 kinase activity.

Besides being useful for human treatment, the compounds of the present invention may also be useful for veterinary treatment of animals including mammals such as horses, cattle, sheep, pigs, dogs, and cats.

### Pharmaceutical Compositions of the Invention

For use in therapy, compounds of the present invention are typically in the form of a pharmaceutical composition. The invention therefore relates to a pharmaceutical composition comprising a compound of formula (I), optionally together with one or more other therapeutically active compound(s), together with a pharmaceutically acceptable excipient, vehicle or carrier(s). The excipient must be "acceptable" in the sense of being compatible with the other ingredients of the composition and not deleterious to the recipient thereof.

Conveniently, the active ingredient comprises from 0.0001-99.9% by weight of the formulation.

In the form of a dosage unit, the compound may be administered one or more times a day at appropriate intervals, always depending, however, on the condition of the patient, and in accordance with the prescription made by the medical practitioner. Conveniently, a dosage unit of a formulation contain between 0.001 mg and 1000 mg, preferably between 0.01 mg and 100 mg, such as 0.1-50 mg of a compound of formula (I).

A suitable dosage of the compound of the invention will depend, inter alia, on the age and condition of the patient, the severity of the disease to be treated and other factors well known to the practising physician. The compound may be administered either orally, parenterally, topically, transdermally or interdermally and other routes according to different dosing schedules, e.g. daily, weekly or with monthly intervals. In general a single dose will be in the range from 0.001 to 400 mg/kg body weight. The compound may be administered as a bolus (i.e. the entire daily dosis is administered at once) or in divided doses two or more times a day.

In the context of topical treatment it may be more appropriate to refer to a "usage unit", which denotes a single dose which is capable of being administered to a patient, and which may be readily handled and packed, remaining as a physically and chemically stable unit dose comprising either the active material as such or a mixture of it with solid, semisolid or liquid pharmaceutical diluents or carriers.

The term "usage unit" in connection with topical use means a unitary, i.e. a single dose, capable of being administered topically to a patient in an application per square centimetre of the treatment area of from 0.001 microgram to 1 mg and preferably from 0.05 microgram to 0.5 mg of the active ingredient in question.

It is also envisaged that in certain treatment regimes, administration with longer intervals, e.g. every other day, every week, or even with longer intervals may be beneficial.

If the treatment involves administration of another therapeutically active compound it is recommended to consult Goodman & Gilman's The Pharmacological Basis of Therapeutics, 9th Ed., J.G. Hardman and L.E. Limbird (Eds.), McGraw-Hill 1995, for useful dosages of said compounds.

The administration of a compound of the present invention with one or more other active compounds may be either concomitantly or sequentially.

The formulations include e.g. those in a form suitable for oral, rectal, parenteral (including subcutaneous, intraperitoneal, intramuscular, intraarticular and intravenous), transdermal, intradermal, ophthalmic, topical, nasal, sublingual or buccal administration.

The formulations may conveniently be presented in dosage unit form and may be prepared by but not restricted to any of the methods well known in the art of pharmacy, e.g. as disclosed in Remington, The Science and Practice of Pharmacy, 21ed ed., 2005. All methods include the step of bringing the active ingredient into association with the carrier, which constitutes one or more accessory ingredients. In general, the formulations are prepared by uniformly and intimately bringing the active ingredient into association with a liquid carrier, semisolid carrier or a finely divided solid carrier or combinations of these, and then, if necessary, shaping the product into the desired formulation.

Formulations of the present invention suitable for oral and buccal administration may be in the form of discrete units as capsules, sachets, tablets, chewing gum or lozenges, each containing a predetermined amount of the active ingredient; in the form of a powder, granules or pellets; in the form of a solution or a suspension in an aqueous liquid or non-aqueous liquid; or in the form of a gel, a nano- or microemulsion, an oil-in-water emulsion, a water-in-oil emulsion or other dispensing systems. Suitable dispersing or suspending agents for aqueous suspensions include synthetic or natural surfactants and viscosifying agents. The active ingredients may also be administered in the form of a bolus, electuary or paste.

A tablet may be made by compressing, moulding or freeze drying the active ingredient optionally with one or more accessory ingredients. Compressed tablets may be prepared by compressing, in a suitable machine, the active ingredient(s) in a free-flowing form such as a powder or granules, optionally mixed by a binder and/or filler; a lubricant; a disintegrating agent such or a dispersing agent. Moulded tablets may be made by moulding, in a suitable machine, a mixture of the powdered active ingredient and suitable carrier moistened with an inert liquid diluent. Freeze dried tablets may be formed in a freeze-dryer from a solution of the drug substance. A suitable filler can be included.

Formulations for rectal administration may be in the form of suppositories in which the compound of the present invention is admixed with low melting point, water soluble or insoluble solids.

Formulations suitable for parenteral administration conveniently comprise a sterile oily or aqueous preparation of the active ingredients, which is preferably isotonic with the blood of the recipient, e.g. isotonic saline, isotonic glucose solution or buffer solution. Furthermore, the formulation may contain co-solvent, solubilising agent and/or complexation agents. The formulation may be conveniently sterilised by for instance filtration through a bacteria retaining filter, addition of sterilising agent to the formulation, irradiation of the formulation or heating of the formulation. Liposomal formulations as disclosed in *e.g*. Encyclopedia of Pharmaceutical Technology, vol.9, 1994, are also suitable for parenteral administration.

Alternatively, the compounds of formula (I) may be presented as a sterile, solid preparation, *e.g*. a freeze-dried powder, which is readily dissolved in a sterile solvent immediately prior to use.

Transdermal formulations may be in the form of a plaster, patch, microneedles, liposomal or nanoparticulate delivery systems or other cutaneous formulations applied to the skin.

Formulations suitable ophthalmic administration may be in the form of a sterile aqueous preparation of the active ingredients, which may be in microcrystalline form, for example, in the form of an aqueous microcrystalline suspension. Liposomal formulations or biodegradable polymer systems e.g. as disclosed in Encyclopedia of Pharmaceutical Technology, vol.2, 1989, may also be used to present the active ingredient for ophthalmic administration.

Formulations suitable for topical, such as dermal, intradermal or ophthalmic administration include liquid or semi-solid preparations such as liniments, lotions, gels, applicants, sprays, foams, film-forming systems, microneedles, micro- or nanoemulsions, oil-in-water or water-in-oil emulsions such as creams, ointments or pastes; or solutions or suspensions such as drops.

For topical administration, the compound of formula (I) may typically be present in an amount of from 0.001 to 20% by weight of the composition, such as 0.01% to about 10 %, but may also be present in an amount of up to about 100% of the composition.

Formulations suitable for nasal or buccal administration include powder, self-propelling and spray formulations, such as aerosols and atomisers. Such formulations are disclosed in greater detail in e.g. Modern Pharmaceutics, 2nd ed., G.S. Banker and C.T. Rhodes (Eds.), page 427-432, Marcel Dekker, New York; Modern Pharmaceutics, 3th ed., G.S. Banker and C.T. Rhodes (Eds.), page 618-619 and 718-721, Marcel Dekker, New York and Encyclopedia of Pharmaceutical Technology, vol. 10, J. Swarbrick and J.C. Boylan (Eds), page 191-221, Marcel Dekker, New York.

In addition to the aforementioned ingredients, the formulations of a compound of formula (I) may include one or more additional ingredients such as diluents, buffers, flavouring agents, colourant, surface active agents, thickeners, penetration enhancing agents, solubility enhancing agents preservatives, e.g. methyl hydroxybenzoate (including anti-oxidants), emulsifying agents and the like.

### METHODS OF PREPARATION

The compounds of the present invention can be prepared in a number of ways well known to those skilled in the art of synthesis. The compounds of formula (I) may for example be prepared using the reactions and techniques outlined below together with methods known in the art of synthetic organic chemistry, or variations thereof as appreciated by those skilled in the art. Preferred methods include, but are not limited to, those described below. The reactions are carried out in solvents appropriate to the reagents and materials employed and suitable for the transformations being effected. Also, in the synthetic methods described below, it is to be understood that all proposed reaction conditions, including choice of solvent, reaction atmosphere, reaction temperature, duration of experiment and work-up procedures, are chosen to be conditions of standard for that reaction, which should be readily recognized by one skilled in the art. Not all compounds falling into a given class may be compatible with some of the reaction conditions required in some of the methods described. Such restrictions to the substituents which are compatible with the reaction conditions will be readily apparent to one skilled in the art and alternative methods can be used. The compounds of the present invention or any intermediate may be purified if required using standard methods well known to a synthetic organist chemist, e.g. methods described in "Purification of Laboratory Chemicals", 6^{th} ed. 2009, W. Amarego and C. Chai, Butterworth-Heinemann. Starting materials are either known compounds, commercially available, or they may be prepared by routine synthetic methods well known to a person skilled in the art.

### General procedures, preparations and examples

Starting materials were commercially available or known in the literature. Reagents and solvents were commercially available and were used without purification unless otherwise noted. Chromatographic purification was performed using a Grace REVELERIS^{®} system with pre-packed REVELERIS^{®} Silica Flash Cartridges, or a Teledyne Isco CombiFlash^{®} Rf system, or manually using silica gel 60. ¹H NMR spectra were recorded on Bruker instruments at 300, 400, or 600 MHz with tetramethylsilane (δ = 0.00 ppm) as internal standard.

The following abbreviations have been used throughout:
- AcOH: acetic acid
- DCM: dichloromethane
- DIPEA: *N*,*N*-diisopropylethylamine
- DMF: *N*,*N*-dimethylformamide
- DMSO: dimethyl sulfoxide
- Et: ethyl
- EtOAc: ethyl acetate
- EtOH: ethanol
- HPLC: high-performance liquid chromatography
- HRMS: high resolution mass spectrum
- MeCN: acetonitrile
- MeOH: methanol
- min: minute(s)
- MS: mass spectrometry or mass spectrum
- NMR: nuclear magnetic resonance spectroscopy
- rt: room temperature, *i.e.* 18-30 °C and typically 20 °C
- THF: tetrahydrofuran
- TLC: thin layer chromatography
- t_{R}: retention time
- UPLC: ultra high performance liquid chromatography
- UV: ultraviolet

### Analytical LC-MS

### Method A

UPLC-MS analyses were performed using a Waters Acquity UPLC system with a 2.1 × 50 mm Acquity UPLC^{®} HSS T3 1.8µm column and an Acquity SQ Detector operated in positive ionization electrospray mode. The mobile phases consisted of 0.1% formic acid in an aqueous 10 mM ammonium acetate solution for buffer A and 0.1% formic acid in acetonitrile for buffer B. A binary gradient (A:B 95:5→5:95) over 1.4 min was used with a flow rate of 1.2 mL/min and the column temperature was 60 °C.

### Method B

UPLC-MS analyses with high resolution mass spectra were performed using a Waters Acquity UPLC system with UV detection at 254 nm and a Waters LCT Premier XE high resolution TOF mass spectrometer operated in positive ionization electrospray mode. The same column and mobile phases A and B as in method A were used, but with a slower gradient (A:B 99:1→1:99 over 4.8 min; 0.7 mL/min; column temp. 40 °C).

### Analytical chiral stationary phase HPLC

Chiral stationary phase HPLC analyses were performed using a Waters Acquity UPLC instrument with a PDA detector and a Waters LCT Premier XE mass spectrometer equipped with a Phenomenex Lux^{®} 3 µm Cellulose-2 (250 × 4.6 mm) column. Isocratic conditions with a mobile phase consisting of 10 mM aqueous ammonium bicarbonate:MeCN 70:30 and a flow rate of 1 mL/min were used. The column was kept at room temperature.

### INTERMEDIATES

### Intermediate 1 2-[trans-4-[(5-Amino-2-chloro-pyrimidin-4-yl)amino]cyclohexyl]acetonitrile

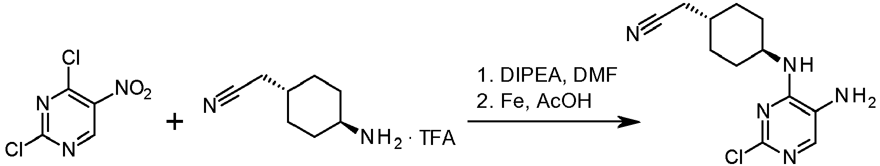

A solution 2,4-dichloro-5-nitropyrimidine (5.0 g, 26 mmol) in DMF (30 mL) was cooled in an ice bath and trans-4-(cyanomethyl)cyclohexylammonium trifluoroacetate (Li, Y.-L. et al. US-20140121198) (6.5 g, 26 mmol) and DIPEA (5.5 mL, 31 mmol) were added. The mixture was stirred at 0 °C for 2 h and then at rt overnight. Volatiles were evaporated and the residue was partitioned between EtOAc (200 mL) and sat. aq. NaHCO₃ solution (200 mL). The layers were separated and the aqueous layer was extracted three times with EtOAc. The combined organic layers were dried over MgSO₄, filtered and evaporated. The residue was dissolved in acetic acid (5 mL) and iron dust (14 g, 260 mmol) was added in portions over 10 min. The mixture was stirred at rt for 2 h after which it was filtered and volatiles were evaporated. The residue was purified by chromatography (120 g pre-packed silica gel column gradient eluted with DCM:MeOH 100:0 to 50:50) to afford the title compound (1.2 g) sufficiently pure for use in the following steps.

UPLC-MS (Method A): t_{R} = 0.53 min, *m*/*z* = 266.1 (M+H⁺).

### EXAMPLES

### Example 1

### 2-[trans-4-[8-[(1R)-1-Hydroxyethyl]-2-(methylamino)purin-9-yl]cyclohexyl]acetonitrile (Compound 1)

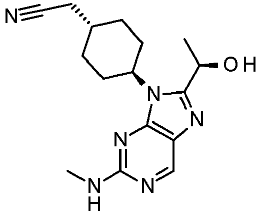

### Step 1 2-[trans-4-[2-Chloro-8-[(1R)-1-hydroxyethyl]purin-9-yl]cyclohexyl]acetonitrile

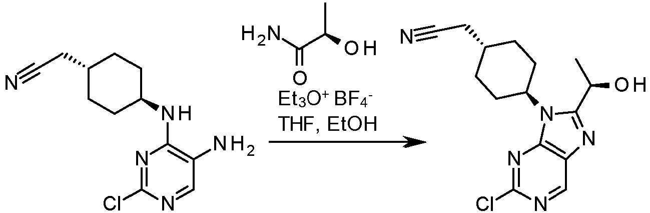

To a solution of triethyloxonium tetrafluoroborate (4.3 g, 23 mmol) in THF (20 mL) under argon was added (R)-lactamide (2.0 g, 23 mmol). The resulting solution was stirred at rt for 2 h and was then added to a solution of Intermediate 1 (1.2 g, 4.5 mmol) in anhydrous ethanol (40 mL). The mixture was stirred at 80 °C overnight. To effect complete conversion, a second portion of triethyloxonium tetrafluoroborate (2.6 g, 14 mmol) was dissolved in THF (15 mL) under argon and (R)-lactamide (1.2 g, 14 mmol) was added. The resulting solution was stirred at rt for 2 h and was added to the reaction mixture above. The mixture was again stirred at 80 °C overnight, volatiles were evaporated and the residue was purified by chromatography (80 g pre-packed silica gel column gradient eluted with DCM:MeOH 100:0 to 90:10) to afford impure product (1.9 g). This was triturated with ether to give the title compound (308 mg, 21%) as a brown solid.
UPLC-MS (Method A): t_{R} = 0.56 min, m/z = 320.2 (M+H⁺).
¹H NMR (600 MHz, DMSO-*d*₆) δ 9.01 (s, 1H), 5.92 (d, *J* = 6.9 Hz, 1H), 5.13 (p, *J* = 6.6 Hz, 1H), 4.66 (tt, *J* = 12.3, 3.7 Hz, 1H), 2.55 (d, *J* = 6.2 Hz, 2H), 2.53 - 2.38 (m, 2H), 2.02 - 1.88 (m, 4H), 1.85 - 1.74 (m, 1H), 1.59 (d, *J* = 6.5 Hz, 3H), 1.36 - 1.25 (m, 2H).

### Step 2 2-[trans-4-[8-[(1R)-1-Hydroxyethyl]-2-(methylamino)purin-9-yl]cyclohexyl]acetonitrile

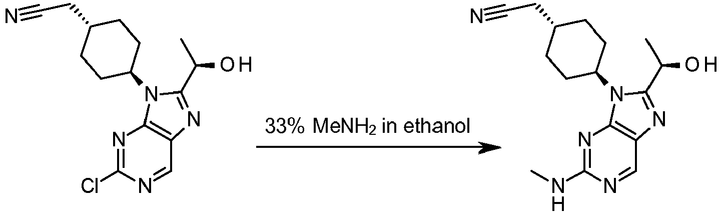

A screwcap vial was charged with the product of Step 1 (300 mg, 0.94 mmol) and 33% methylamine in ethanol (5 mL) and the mixture was shaken at 70 °C for 3 h. Volatiles were evaporated and the residue was purified by chromatography (24 g pre-packed silica gel column gradient eluted with DCM:MeOH 100:0 to 90:10) to afford the title compound (200 mg, 68%).
UPLC-MS (Method B): t_{R} = 1.79 min, m/z = 315.1811 (M+H⁺).
Analytical chiral stationary phase HPLC: t_{R} = 10.1 min. No other peaks were detected. The (S) enantiomer is expected at t_{R} = 11.0 min, see Example 2.
¹H NMR (600 MHz, DMSO-*d*₆) δ 8.52 (s, 1H), 6.83 (br s, 1H), 5.63 (d, *J* = 6.7 Hz, 1H), 4.95 (p, *J* = 6.5 Hz, 1H), 4.48 (tt, *J* = 12.0, 4.0 Hz, 1H), 2.82 (d, *J* = 4.7 Hz, 3H), 2.69 - 2.56 (m, 2H), 2.54 (d, *J* = 6.3 Hz, 2H), 1.95 - 1.80 (m, 4H), 1.76 - 1.67 (m, 1H), 1.53 (d, *J* = 6.5 Hz, 3H), 1.32 - 1.21 (m, 2H).

### Example 2

### 2-[trans-4-[8-(1-Hydroxyethyl)-2-(methylamino)purin-9-yl]cyclohexyl]acetonitrile (Compound 2)

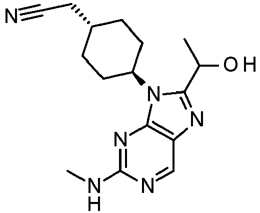

Prepared as Example 1 but starting from racemic lactamide.
UPLC-MS (Method B): t_{R} = 1.79 min, *m*/*z* = 315.1894 (M+H⁺).
Analytical chiral stationary phase HPLC: (R) enantiomer t_{R} = 10.2 min; (*S*) enantiomer t_{R} = 11.0 min. The absolute configurations were established by comparison with a sample of the (*R*) enantiomer (t_{R} = 10.1 min) prepared from enantiomerically enriched (*R*)-lactamide, see Example 1.
¹H NMR (300 MHz, DMSO-*d*₆) δ 8.52 (s, 1H), 6.82 (br q, *J* = 4.0 Hz, 1H), 4.96 (q, *J* = 6.5 Hz, 1H), 4.57 - 4.40 (m, 1H), 2.83 (d, *J* = 4.7 Hz, 3H), 2.74 - 2.56 (m, 2H), 2.54 (d, *J* = 6.3 Hz, 2H), 1.99 - 1.79 (m, 4H), 1.78 - 1.64 (m, 1H), 1.53 (d, *J* = 6.5 Hz, 3H), 1.36 - 1.17 (m, 2H).

### Example 3

### 2-[trans-4-[8-[(1R)-1-Hydroxyethyl]-2-(trideuteriomethylamino)purin-9-yllcyclohexyllacetonitrile (Compound 3)

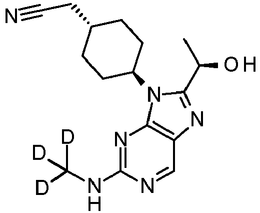

A screwcap vial charged with 2-[trans-4-[2-chloro-8-[(1R)-1-hydroxyethyl]purin-9-yl]cyclohexyl]acetonitrile (10 mg, 31 µmol), methyl-3D-amine hydrochloride (22 mg, 313 µmol), DIPEA (56 µL, 313 µmol) and isopropanol (0.2 mL) was shaken overnight at 120 °C. Acid prep-HPLC purification follow by freeze-drying afforded the title compound. UPLC-MS (Method B): t_{R} = 1.79 min, *m*/*z* = 318.214 (M+H⁺).

¹H NMR (600 MHz, DMSO-*d*₆) δ 8.52 (s, 1H), 6.80 (br s, 1H), 5.64 (br s, 1H), 4.99 - 4.92 (m, 1H), 4.48 (tt, *J* = 12.1, 4.1 Hz, 1H), 2.71 - 2.56 (m, 2H), 2.54 (d, *J* = 6.3 Hz, 2H), 1.96 - 1.80 (m, 4H), 1.76 - 1.67 (m, 1H), 1.53 (d, *J* = 6.5 Hz, 3H), 1.26 (qt, *J* = 4.3, 12.8 Hz, 2H).

### JAK kinase assays

Human baculovirus-expressed Janus kinase (JAK) 1, 2, 3 and tyrosin kinase (TYK) 2 were purchased from Carna Biosciences, Inc (#08-144, -045, -046, -147 resp.). All four purified enzymes contain only the catalytic domain. JAK1 (aa 850-1154) and TYK2 (aa 871-1187) are expressed with an N-terminally fused GST-tag, and JAK2 and JAK3 with an N-terminally fused His-tag. Inhibition of phosphorylation of a synthetic peptide was measured in an HTRF-based assay (CisBio #62TK0PEC). First, 75 nL of test compound solution (100% DMSO) was added to a white shallow 384-well plate (NUNC #264706) using a Labcyte ECHO 550 liquid handler. Thereafter, 1 µL of compound dilution buffer (50 mM HEPES, 0.05% bovine serum albumin) and 2 µL of TK solution (TK substrate-biotin in kinase buffer [1x enzymatic buffer from HTRFKinEASE TK kit, 1 mM DTT]) was added. Then, 5 µL kinase-ATP mix (prepared in kinase buffer) was added to the wells and the plates were incubated at RT for 20 (JAK2, 3 and TYK2) to 40 (JAK1) min. For all four kinases a concentration of ATP that corresponded to the Kₘ for ATP was used. The final concentrations of buffers, substrate, kinase and ATP were: JAK1: 50 mM Hepes buffer pH 7.0, 0.01% BSA, 10 mM MgCl₂, 1 mM DTT, 7 µM ATP, 50 nM SEB, 1 µM TK Substrate-Biotin and 5 ng JAK1; JAK2: 50mM Hepes buffer pH 7.0, 0.01% BSA, 5 mM MgCl₂, 1 mM DTT, 4 µM ATP, 1 µM TK Substrate-Biotin and 0.1 ng JAK2; JAK3: 50 mM Hepes buffer pH 7.0, 0.01% BSA, 5 mM MgCl₂, 1 mM DTT, 2 µM ATP, 1 µM TK Substrate-Biotin and 0.3 ng JAK3; TYK2: 50 mM Hepes buffer pH 7.0, 0.01% BSA, 5 mM MgCl₂, 1 mM DTT, 13 µM ATP, 50 nM SEB, 1 µM TK Substrate-Biotin and 0.8 ng TYK2. Thereafter, the kinase reaction was stopped by adding 4 µL detection mix (final concentrations: 50mM Hepes buffer pH 7.0, 0.01% BSA, 0.8 M KF, 20 mM EDTA, 42 nM Streptavidin-XL665 and 1:400 STK Ab Cryptate) and the plates were incubated overnight in the dark. A PerkinElmer Envision reader was used to quantify the HTRF signal using the following filters; 320 nm excitation filter, 665 nm emission filter and a 615 nm 2^{nd} emission filter. A ratio ((665/615) × 10⁴) was calculated for each well.

### STAT6 assay

Twenty-five µL of a STAT6 bla-RA1 (Invitrogen #K1243) cell suspension was seeded with a density of 30-40,000 cells/well in 384-well Black View-plates (PerkinElmer #6007460) with clear bottom in assay medium (Opti-MEM (Invitrogen #11058-021) + 0.5% heat inactivated fetal bovine Serum (Invitrogen #10082-147) + 1% non-essential amino acids (Invitrogen #11140-050) + 1% sodium pyruvate (Invitrogen #11360-070) + 1% penicillin/streptomycin (Invitrogen #15140-122)); containing 550 ng/mL of CD40 ligand (Invitrogen #PHP0025). The cell plates were incubated overnight in a humidified 37 °C air/CO2 (95%/5%) incubator. The following day, 125 nL of solutions of test compounds and reference compounds were transferred to cell plates using the Labcyte Echo 550 liquid handler. The plates were then incubated for 1 h in a humidified 37 °C air/CO2 (95%/5%) incubator. Hereafter recombinant human interleukin 4 (Invitrogen #PHC0045) were added to the plates also using the Labcyte Echo 550 to a final concentration of 10 ng/mL. The cells were then incubated for 4½-5 h in a humidified 37 °C air/CO2 (95%/5%) incubator. 8 µL of LiveBLAzer substrate mixture (Invitrogen #K1095) were then added to the assay plates, which were incubated overnight at RT. Fluorescence was then measured: Excitation: 405 nm; Emission: 460 nm (green channel), Emission: 535 nm (blue channel). Background was subtracted in both emission channels and the ratio 460/535 nm was calculated for each well.

### STAT5 assay

Twenty-five µL of a STAT5 irf1-bla TF1 (Invitrogen #K1219) cell suspension was seeded with a density of about 10,000 cells/well in 384-well Black View-plates (PerkinElmer #6007460) with clear bottom in assay medium (Opti-MEM (Invitrogen #11058-021) + 0.5% heat inactivated fetal bovine Serum (Invitrogen #10082-147) + 1% non-essential amino acids (Invitrogen #11140-050) + 1% sodium pyruvate (Invitrogen #11360-070) + 1% penicillin/streptomycin (Invitrogen #15140-122)). The cell plates were incubated overnight in a humidified 37 °C air/CO2 (95%/5%) incubator. The following day, 125 nL of solutions of test compounds and reference compounds were transferred to cell plates using the Labcyte Echo 550 liquid handler. The plates were then incubated for 1 h in a humidified 37 °C air/CO2 (95%/5%) incubator. Hereafter recombinant human erythropoietin (EPO) (Invitrogen #PHC9634) was added to the plates also using the Labcyte Echo 550 to a final concentration of 10 ng/mL. The cells were then incubated for 4½-5 h in a humidified 37 °C air/CO2 (95%/5%) incubator. 8 µL of LiveBLAzer substrate mixture (Invitrogen #K1095) were then added to the assay plates, which were then incubated overnight at RT. Fluorescence was then measured: Excitation: 405 nm; Emission: 460 nm (green channel), Emission: 535 nm (blue channel). Background was subtracted in both emission channels and the ratio 460/535 nm was calculated for each well.

Compounds of the present invention were tested in JAK kinase assays, STAT 5 and STAT6 assays. Results are listed in Table 1.

**Table 1**

| Compound | JAK1 EC₅₀ (nM) | JAK2 EC₅₀ (nM) | JAK3 EC₅₀ (nM) | TYK2 EC₅₀ (nM) | STAT5 EC₅₀ (nM) | STAT6 EC₅₀(nM) |
|---|---|---|---|---|---|---|
| 1 | 5 | 157 | 602 | 19 | 5190 | 146 |
| 2 | 9 | 274 | 798 | 47 | 12800 | 213 |
| 3 | 3 | 129 | 510 | 13 | 4870 | 142 |

## Claims

1. A compound according to general formula (I) 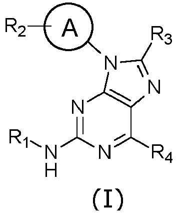 wherein
A represents C₆-cycloalkyl;
R₁ represents C₁-alkyl, wherein said C₁-alkyl is optionally substituted with one or more substituents selected from the group consisting of deuterium;
R₂ represents C₁-alkyl, wherein said C₁-alkyl is substituted with a substituent selected from R₅, and wherein said C₁-alkyl is optionally substituted with one or more deuterium;
R₃ represents C₂-alkyl, wherein said C₂-alkyl is substituted with a substituent selected from R₆ and wherein said C₂-alkyl is optionally substituted with one or more deuterium;
R₄ represents hydrogen or deuterium;
R₅ represents cyano;
R₆ represents hydroxyl;
or pharmaceutically acceptable salts, hydrates or solvates thereof.

2. The compound according to claim 1 selected from
2-[trans-4-[8-[(1R)-1-Hydroxyethyl]-2-(methylamino)purin-9-yl]cyclohexyl]acetonitrile,
2-[trans-4-[8-(1-Hydroxyethyl)-2-(methylamino)purin-9-yl]cyclohexyl]acetonitrile,
2-[trans-4-[8-[(1R)-1-Hydroxyethyl]-2-(trideuteriomethylamino)purin-9-yl]cyclohexyl]acetonitrile
or pharmaceutically acceptable salts, hydrates or solvates thereof.

3. A compound according to any one of claims 1-2 for use as a medicament.

4. A compound according to any one of claims 1-2 for use in the prophylaxis and/or treatment of diseases of the immune system such as autoimmune diseases, or of diseases related to deregulation of the immune system.

5. The compound for use according to claim 4 in the prophylaxis and/or treatment of diseases selected from psoriasis and atopic dermatitis

6. A pharmaceutical composition comprising a compound according to any one of claims 1-2 together with a pharmaceutically acceptable vehicle or excipient or pharmaceutically acceptable carrier(s).

7. The pharmaceutical composition according to claim 6 together with one or more other therapeutically active compound(s).

8. The compound according to any one of claims 1-2 for use in the treatment of a disease, selected from psoriasis, atopic dermatitis, scleroderma, rosacea, skin cancers, dermatitia, dermatitis herpetiformis, dermatomyositis, vitiligo, alopecia areata, contact dermatitis, eczema, xerosis, ichthyosis, urticaria and chronic idiophatic pruritus; respiratory diseases like asthma, chronic obstructive pulmonary disease, pulmonary fibrosis, cystic fibrosis, rhinitis, bronchiolitis, byssinosis, pneumoconiosis, bronchiectasis, hypersensitivity pneumonitis, lung cancers, mesothelioma and sarcoidosis; gastrointestinal diseases like inflammatory bowel disease, ulcerative colitis, Crohn's disease, retroperitoneal fibrosis, celiac disease and cancers; eye diseases like myasthenia gravis, Sjogren's syndrome, conjunctivitis, scleritis, uveitis, dry eye syndrome, keratitis, iritis; systemic indications like lupus, multiple sclerosis, rheumatoid arthritis, type I diabetes and complications from diabetes, 10 cancers, ankylosing spondylitis and psoriatic arthritis.

9. The compound according to claim 8 for use in the treatment of psoriasis.

10. The compound for use according to any one of claims 8-9 in combination with other therapeutically active compounds.

## Patentansprüche

1. Verbindung nach allgemeiner Formel (I) 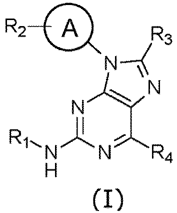 wobei A für C₆-Cycloalkyl steht;
R₁ für C₁-Alkyl steht, wobei das C₁-Alkyl optional mit einem oder mehreren Substituenten substituiert ist, ausgewählt aus der Gruppe bestehend aus Deuterium;
R₂ für C₁-Alkyl steht, wobei das C₁-Alkyl mit einem Substituenten, ausgewählt aus R₅ substituiert ist, und wobei das C₁-Alkyl optional mit einem oder mehr Deuterium substituiert ist;
R₃ für C₂-Alkyl steht, wobei das C₂-Alkyl mit einem Substituenten, ausgewählt aus R₆, substituiert ist, und wobei das C₂-Alkyl optional mit einem oder mehr Deuterium substituiert ist;
R₄ für Wasserstoff oder Deuterium steht;
R₅ für Cyano steht;
R₆ für Hydroxyl steht;
oder für pharmazeutisch verträgliche Salze, Hydrate oder Solvate davon steht.

2. Verbindung nach Anspruch 1, ausgewählt aus
2-[Trans-4-[8-[(1*R*)-1-Hydroxyethyl]-2-(methylamino)purin-9-yl]cyclohexyl]acetonitril,
2-[Trans-4-[8-(1-Hydroxyethyl)-2-(methylamino)purin-9-yl]cyclohexyl]acetonitril,
2-[Trans-4-[8-[(1*R*)-1-Hydroxyethyl]-2-(trideuteriomethylamino)purin-9-yl]cyclohexyl]acetonitril
oder pharmazeutisch verträglichen Salzen, Hydraten oder Solvaten davon.

3. Verbindung nach einem der Ansprüche 1 bis 2 zur Verwendung als Medikament.

4. Verbindung nach einem der Ansprüche 1 bis 2 zur Verwendung bei der Prophylaxe und/oder Behandlung von Erkrankungen des Immunsystems, wie Autoimmunerkrankungen, oder von Erkrankungen, die sich auf die Deregulation des Immunsystems beziehen.

5. Verbindung zur Verwendung nach Anspruch 4 bei der Prophylaxe und/oder Behandlung von Erkankungen, ausgewählt aus Psoriasis und atopischer Dermatitis.

6. Pharmazeutische Zusammensetzung, umfassend eine Verbindung nach einem der Ansprüche 1 bis 2 zusammen mit einer pharmazeutisch verträglichen Trägersubstanz oder einem pharmazeutisch verträglichen Hilfsstoff oder pharmazeutisch verträglichen Träger(n).

7. Pharmazeutische Zusammensetzung nach Anspruch 6 zusammen mit einer oder mehreren anderen therapeutisch wirksamen Verbindung(en).

8. Verbindung nach einem der Ansprüche 1 bis 2 zur Verwendung bei der Behandlung einer Erkrankung, ausgewählt aus Psoriasis, atopischer Dermatitis, Sklerodermie, Rosacea, Hautkrebs, Dermatitia, Dermatitis herpetiformis, Dermatomyositis, Vitiligo, Alopezie areata, Kontaktdermatitis, Ekzem, Xerose, Ichthyosis, Urticaria und chronisch idiophatischem Pruritus; Atemwegserkrankungen, wie Asthma, chronisch obstruktiver Lungenerkrankung, pulmonaler Fibrose, zystischer Fibrose, Rhinitis, Bronchitis, Byssinose, Pneumokoniose, Bronchiektase, Hypersensitivitätspneumonitis, Lungenkrebs, Mesotheliom und Sarkoidose; gastrointestinalen Erkrankungen, wie entzündlichen Darmerkrankungen, Colitis ulcerosa, Morbus Crohn, retroperitonealer Fibrose, Zöliakie und Krebs; Augenerkrankungen, wie Myasthenia gravis, Sjögren-Syndrom, Konjunktivitis, Skleritis, Uveitis, Sicca-Syndrom, Keratitis, Iritis; systemischen Indikationen, wie Lupus, multipler Sklerose, rheumatoider Arthritis, Typ-I-Diabetes und Komplikationen aufgrund von Diabetes, 10 Krebsarten, Spondylitis ankylosans und Psoriasis-Arthritis.

9. Verbindung nach Anspruch 8 zur Verwendung bei der Behandlung von Psoriasis.

10. Verbindung zur Verwendung nach einem der Ansprüche 8 bis 9 in Kombination mit anderen therapeutisch wirksamen Verbindungen.

## Revendications

1. Composé selon la formule générale (I) 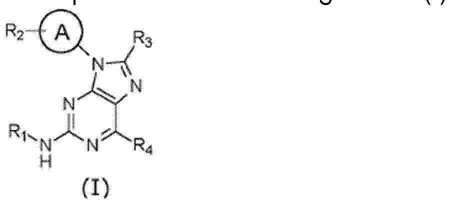 dans laquelle
A représente cyclo-alkyle en C₆ ;
R₁ représente alkyle en C₁, ledit alkyle en C₁ étant éventuellement substitué par un ou plusieurs substituants choisis dans le groupe consistant en deutérium ;
R₂ représente alkyle en C₁, ledit alkyle en C₁ étant substitué par un substituant choisi parmi R₅, et ledit alkyle en C₁ étant éventuellement substitué par un ou plusieurs deutériums ;
R₃ représente alkyle en C₂, ledit alkyle en C₂ étant substitué par un substituant choisis parmi
R₆, et ledit alkyle en C₂ étant éventuellement substitué par un ou plusieurs deutériums ;
R₄ représente l'hydrogène ou le deutérium ;
R₅ représente cyano ;
R₆ représente hydroxyle ;
ou des sels pharmaceutiquement acceptables, hydrates ou solvates de ceuxci.

2. Composé selon la revendication 1, choisi parmi
2-[*trans*-4-[8-[(1R)-1-Hydroxyéthyl]-2-(méthylamino)purin-9-yl]cyclohexyl]acé-tonitrile,
2-[frans-4-[8-(1-Hydroxyéthyl]-2-(méthylamino)purin-9-yl]cyclohexyl]acétoni-trile,
2-[*trans-*4-[84(1R)-1-Hydroxyéthyl]-2-(trideuteriométhylamino)purin-9-yl]cyclohexyl]acétonitrile
ou des sels pharmaceutiquement acceptables, hydrates ou solvates de ceuxci.

3. Composé selon l'une quelconque des revendications 1 à 2 pour l'utilisation en tant que médicament.

4. Composé selon l'une quelconque des revendications 1 à 2 destiné à être utilisé dans la prophylaxie et / ou le traitement de maladies du système immunitaire telles que des maladies auto-immunes, ou de maladies liées à la dérégulation du système immunitaire.

5. Composé destiné à être utilisé selon la revendication 4 dans la prophylaxie et / ou le traitement de maladies choisies parmi le psoriasis et la dermatite atopique.

6. Composition pharmaceutique, comprenant un composé selon l'une quelconque des revendications 1 à 2 conjointement avec un véhicule ou un excipient pharmaceutiquement acceptable ou un ou des supports pharmaceutiquement acceptables.

7. Composition pharmaceutique selon la revendication 6 avec un ou plusieurs autre(s) composé(s) thérapeutiquement actif(s).

8. Composé selon l'une quelconque des revendications 1 à 2 destiné à être utilisé dans le traitement d'une maladie choisie parmi le psoriasis, la dermatite atopique, la sclérodermie, la rosacée, les cancers de la peau, la dermatite, la dermatite herpétiforme, la dermatomyosite, le vitiligo, l'alopécie areata, la dermatite de contact, l'eczéma, la xérose, l'ichtyose, l'urticaire et le prurit idiophatique chronique ; les maladies respiratoires telles que l'asthme, la bronchopneumopathie chronique obstructive, la fibrose pulmonaire, la mucoviscidose, la rhinite, la bronchiolite, la byssinose, la pneumoconiose, la bronchiectasie, la pneumonie d'hypersensibilité, les cancers du poumon, le mésothéliome et la sarcoïdose ; les maladies gastro-intestinales telles que la maladie intestinale inflammatoire, la colite ulcéreuse, la maladie de Crohn, la fibrose péritonéale, la maladie coeliaque et les cancers ; les maladies oculaires telles que la myasthénie grave, le syndrome de Sjögren, la conjonctivite, la sclérite, l'uvéite, le syndrome de l'oeil sec, la kératite, l'iritis ; des indications systémiques telles que le lupus, la sclérose en plaques, la polyarthrite rhumatoïde, le diabète de type 1 et les complications du diabète, des cancers, de la spondylarthrite ankylosante et de l'arthrite psoriasique.

9. Composé selon la revendication 8 destiné à être utilisé dans le traitement du psoriasis.

10. Composé destiné à être utilisé selon l'une quelconque des revendications 8 à 9 en combinaison avec d'autres composés thérapeutiquement actifs.
